# EUROPEAN PATENT APPLICATION

(11) **EP 1 314 430 A1**
(43) Date of publication of application: **28.05.2003**
(21) Application number: 02019356.1
(22) Date of filing: 27.01.1997
(51) Int. Cl.: A61K 35/76

(54) **Treatment of non-neuronal cancer using HSV mutant**

(30) Priority: 25.01.1996 GB 9601507; 09.11.1996 GB 9623365
(62) Divisional of application: 97901695.3
(71) Applicant: THE UNIVERSITY COURT OF THE UNIVERSITY OF GLASGOW, Glasgow G12 8QQ (GB); THE WISTAR INSTITUTE, Philadelphia, PA 19104-4268 (US)
(72) Inventor: Brown, Moira Susanne, Neurovirology Research Lab., Glasgow, G51 4TF (GB); Maclean, Roderick Alasdair, Glasgow, G11 5JR (GB); Fraser, William Nigel, Philadelphia, PA 19104-4268 (US); Randazzo, Paul Bruce, University of Pennsylvania, Philadelphia, PA 19104 (US); Albelda, Steven, University of Pennsylvania, Philadelphia, PA 19104 (US); Kaiser, Larry, University of Pennsylvania, Philadelphia, PA 19104 (US)
(74) Representative: Cripps, Joanna Elizabeth

(57) **Abstract**

A mutant herpes simplex virus which,has been modified in the γ 34.5 gene such that the gene is non-functional is used to treat a non-neuronal cancer such as a mesothelioma, ovarian carcinoma, bladder cancer or melanoma. Typically, the mutant herpes simplex virus has been modified within the BamHI restriction fragment of the long terminal repeat of the viral genome.

## Description

### FIELD OF INVENTION

The present invention relates to the treatment of non-neuronal cancer tumors, particularly mesotheliomas, melanoma, ovarian carcinoma or bladder cancer whether the tumors are metastatic tumors or primary tumors.

### BACKGROUND OF THE INVENTION

The DNA sequence of herpes simplex type 1 (HSV-1) is known and is linear with a length of about 152k residues. It consists of two covalently linked segments, designated long (L) and short (S). Each segment contains a unique sequence flanked by a pair of inverted terminal repeat sequences. The long repeat (R_{L}) and short repeat (R_{S}) are distinct. The unique long (U_{L}) region includes genes UL1 to UL56, and the U_{S} region includes genes US1 to US12.

The U_{L} region is flanked by a terminal repeat region (TRL) and an internal repeat region (IRL) which lies adjacent the IRS of the U_{S} region. Two genes RL1 and RL2 lie within each of the repeat regions TRL and IRL. The RL1 gene codes for the protein ICP 34.5, and this gene is referred to herein as γ34.5.

A number of naturally occurring and artificially-engineered HSV-1 mutants have recently been identified that appear to replicate preferentially in transformed cells (Martuza *et al*, 1991; Mineta *et al*, 1994). Because of the natural tropism of wild type herpes virus for neuronal tissue, the published uses of modified, replicating HSV to treat cancer have centered around tumors of CNS (central nervous system) origin. Initial experiments utilising HSV-1 mutants with deletions in the thymidine kinase gene (HSV-TK⁻) showed dose dependent improvement in the survival of nude mice bearing human gliomas, medulloblastoma, malignant or atypical meningioma and neurofibrosarcoma both *in vitro* and in tumor bearing nude mice (Martuza *et al*, 1991; Markert *et al*, 1993). More recently, additional "replication restricted" non-neurovirulent mutants of HSV that contain the HSV-TK gene (a potential safety factor that would allow elimination of virus by treatment with the drug ganciclovir), but lack other HSV genes have been developed and have shown even more promise in CNS tumors.

A mutant HSV-1 called R3616, containing a 1000 base pair (bp) deletion in γ34.5, with LD₅₀ (minimum dose of virus that kills 50% of infected animals) that is at least 3x10³ fold greater than wild type F strain virus from which it was derived, has been shown to improve the outcome of nude mice bearing intracranial human gliomas (Mineta *et al*, 1995).

In the work presented here, we have utilised an HSV-1 strain 17 mutant virus called 1716, that has a 759 bp deletion in each copy of γ34.5 of the long repeat region (R_{L}). The construction of mutant virus 1716 is described in published International patent application WO 92/13943 (PCT/GB92/00179) the contents of which are incorporated herein by reference. However, this patent publication is solely concerned with the use of mutant 1716 as a vaccine, either in itself or as a vector vaccine which includes a heterologous gene coding for an antigen.

WO 96/03997 (PCT/GB95/01791) includes data showing the efficacy of HSV 1716 against brain tumors.

### SUMMARY OF THE INVENTION

The present invention is based on the surprising discovery that HSV which is γ34.5 null is effective against non-neuronal cancers. Since HSV is known to selectively inhabit the neuronal system (including the peripheral and central nervous system) and where it may remain in a latent state, it was unexpected that an HSV mutant could be effective against cancers of non-neuronal origin. Moreover, replication of the HSV mutant *in vivo* is restricted to the tumor cells so that "normal" non-tumor cells are unaffected.

Accordingly, the present invention provides the use of a mutant herpes simplex virus which has been modified in the γ34.5 gene such that the gene is non-functional, in the manufacture of a medicament for use in treating a non-neuronal cancer.

The invention also relates to a method of treating a non-neuronal cancer in a mammal, which method comprises the step of administering to the mammal an effective amount of a mutant herpes simplex virus which has been modified in the γ34.5 gene such that the gene is non-functional. The invention further provides an agent for treating a non-neuronal cancer, comprising a mutant herpes simplex virus which has been modified in the γ34.5 gene such that the gene is non-functional.

### BRIEF DESCRIPTION OF THE FIGURES

Figure 1 shows an HSV-1716 single step viral growth curve on human malignant mesothelioma cells. Innoculum at time 0 was 5,000 plaque forming units (PFU) of virus (multiplicity of infection, MOI=0.1). At twenty-four hours the amount of virus present had increased by four logs over the initial input innoculum.
Figure 2 shows an MTT assay for human malignant mesothelioma cell viability as a function of time and varying MOI. The % of control growth is the ratio of mean MTT activity in infected cells (n=6 wells at each time point) to the activity in a matched uninfected cells (n=6 wells at each time point).
Figure 3 shows the mean tumor score in animals day 28 animals (tumor/HSV animals received 5 x 10⁶ pfu HSV-1716 on day 14). The mean tumor score in the control group was 3.9 ± 0.1 versus a mean tumor score in the treatment group of 1.4 ± 0.2 (p<0.001);
Figure 4 shows an HSV-1716 viral dose response survival study. SCID mice received 30 x 10⁶ human malignant mesothelioma cells on day 0. Seven days later one animal was sacrificed to confirm tumor. The remaining animals were randomized into four groups: control (n=11, culture media), low dose (n=10, 5 x 10⁴ pfu HSV-1716), middle dose (n=10, 5 x10⁵ pfu HSV-1716), and high dose (n=10, 5 x 10⁶ pfu-1716);
Figure 5 shows virus titer following infection of subconfluent monolayers of 1205 cells with 5,000 PFU of HSV-1716 or HSV17+; and
Figure 6 shows tumor volume of treated and control tumors at various times after viral therapy.

### DETAILED DESCRIPTION OF THE INVENTION

For the purposes of the present invention "non-functional" means that the gene has been modified by deletion, insertion or substitution (or other change in the DNA sequence such as by rearrangement) such that it does not express the normal product or a functionally equivalent product. The effect of the non-functionality of the gene is that the neurovirulence of the virus to the patient is substantially removed. Each of the two γ34.5 genes is non-functional.

Thus the invention relies on the finding that rendering the γ34.5 gene non-functional provides an HSV mutant which is particularly effective in destroying dividing non-neuronal tumor cells, whilst at the same time the HSV mutant does not replicate within normal non-cancerous cells. It therefore has the potential to provide a safe anti-cancer treatment.

Two types of herpes simplex virus are known HSV-1 and HSV-2 and either may be employed in the present invention to provide the HSV mutant. Inter-type recombinants containing DNA from both types could also be used. HSV-1 and HSV-2 mutants 1716, 1771, 2604, 2616 and 2621 are described herein.

The modification may be effected at any convenient point within the γ34.5 gene, and such point generally corresponds to a restriction enzyme site or sites. The modification may be within the BamHI s restriction fragment of each R_{L} terminal repeat (corresponding to 0-0.02 and 0.81 - 0.83 mu). The modification is typically a deletion of 0.1 to 3kb, particularly 0.5 to 2.5kb, and especially 0.7 to 0.8 kb. The simple insertion of a stop codon is also effective in preventing production of the ICP 34.5 protein.

The HSV genome also includes a number of other genes which are non-essential to the successful culturing of the virus. Their removal may further contribute to the safety of the HSV mutant by further reducing neurovirulence and reducing the likelihood of recombination to the wild type. It is, of course, necessary to retain within the HSV mutant the ability to culture the mutant so that the mutant is self-replicating and stocks of the mutant can be grown in tissue culture. Lethal modifications of the genome which remove the ability to culture the HSV mutant are not acceptable, unless the missing gene products can be provided to the culture system in an alternative way e.g. by the use of a complementing cell line containing a plasmid which expresses the missing gene product.

Thus, in addition to the primary modification to the γ34.5 gene of the R_{L} region, it may be advantageous to also include in the HSV mutant one or more secondary modifications which are generally within non-essential genes unless the missing gene product can be provided in an alternative way.

The present invention also encompasses the use of an HSV mutant which includes in addition to the primary modification, a secondary modification (for example within Vmw65). The mutant may be derived from HSV-1 or HSV-2.

In a similar way, other secondary modifications may involve modification of the latency associated transcript (LAT) promoter so as to render the promoter non-functional and prevent transcription thereof.

Herpes simplex virus naturally infects the brain and nervous system. It is therefore surprising that the HSV mutant is effective against tumors outside the brain and nervous system. Such tumors may be metastatic tumors where the cancer cells originate elsewhere or may be primary tumors. The non-neuronal cancer being treated will generally be a primary cancer of non-neuronal cell type, or a secondary cancer of non-neuronal cell type which has metastasised to a non-neuronal (e.g. non-CNS) location in the patient's body.

On the basis of the results presented herein, which surprisingly show the ability of the HSV mutant to combat non-neuronal tumors, it is believed that the anti-tumor effectiveness of the HSV mutant extends to the treatment of non-neuronal (e.g. non-CNS) cancers in general, including the treatment of mesotheliomas, melanoma, ovarian carcinoma, and bladder cancer. The condition of a patient suffering from such a cancer can be improved. The treatment is particularly applicable to primary tumors which are localised, rather than metastatic tumors. The efficacy of treatment according to the invention employing the HSV mutant will depend on the time after origination of the tumor-at which the treatment is initiated, but efficacy is improved by early treatment for example in 1 to 30 days.

The LD₅₀ (minimum dose of virus that kills 50% of infected animals) of the 1716 mutant in respect of mice is 10⁶ fold greater than that of the wild type 17+ virus from which it is derived (for cerebral tumors). Thus the neurovirulence of 1716 is essentially removed relative to the wild type virus.

The effective non-toxic dose of HSV mutant can be determined by routine investigation by the skilled addressee, and will depend on a number of factors including the particular species of mammal and the extent of development of the tumor. A guide can be obtained from the Examples herein, which show that in the mouse relatively high doses (4x10⁶ pfu) can significantly improve survival. Preferred doses for mice are in the range 1x10⁴ to 1x10⁸ particularly 1x10⁶ to 1x10⁷ pfu. The doses for other mammals can be estimated accordingly by the skilled man. For humans doses will generally be in the range 1x10⁶ to 1x10⁸ pfu.

In a further aspect of the invention there is provided a method of treating non-neuronal cancer in mammals, in particular in humans by administering a pharmaceutical formulation comprising the HSV mutant to a mammal, in particular to humans. Thus, the method of treatment can comprise the administration of a pharmaceutical formulation comprising the HSV mutant by injection directly into the tumor, parenterally into the blood stream feeding the tumor or intraperitoneally. The tumor may be surgically removed or debulked prior to treatment with the HSV mutant.

It will usually be presented as a pharmaceutical formulation including a carrier or excipient, for example an injectable carrier such as saline or apyrogenic water. The formulation may be prepared by conventional means.

The following Examples illustrate the invention.

### EXAMPLE 1

### MATERIALS AND METHODS

### Mutants in the HSV RL1 gene

Our laboratory has isolated a number of deletion mutants and point mutants in the RL1 gene of both HSV-1 strain 17 and HSV-2 strain HG52.

### HSV-1 strain 17

### 1716

The genome of this virus has a 759 bp deletion located within each copy of the BamHI fragment (0-0.02 and 0.81-0.83 map units) of the long repeat region of the genome. The deletion removes one complete copy of the 18 bp DR1 element of the 'a' sequence and terminates 1105 bp upstream of the 5' end of IE gene 1. Most of RL1 including the initiating methionine is removed and the mutant fails to make ICP34.5. Following intracerebral inoculation of mice, the LD₅₀ value of 1716 is 7x10⁶/mouse compared to <10 for the parental strain 17+.

Its production is described in published patent application WO 92/13943 (PCT/GB92/00179). The mutant virus was passaged for use in this study by Dr. Nigel N. Fraser (Philadelphia, PA).

### 1771

The genome of this virus has a stop codon functional only in the assigned RL1 reading frame 9 bp downstream from the initiating ATG. The LD₅₀ value of 1771 is >10⁶ PFU/mouse following intracerebral inoculation and its latency phenotype is indistinguishable from 1716. 1771 fails to synthesize ICP34.5.

### HSV-2 strain HG52 mutants.

### 2604

This virus has a deletion of 1488 bp in both long repeats of the genome which extends from the DR1/Ub boundary of the 'a' sequence to 511 bp upstream of the 5' end of IE1. The deletion removes the whole of RL1. 2604 has a LD₅₀ value of >10⁷ PFU/mouse compared to <10² for wild type strain HG52. Although formal proof of lack of synthesis of ICP34.5 has not been obtained due to the unavailability of an antiserum which detects the type 2 protein, the phenotype of the virus *in vivo* has been shown to be unambiguously due to the RL1 deletion.

### 2616

This virus has 786 bp of both copies of RL1 deleted but retains 782 bp upstream of the 5' end of IE1 and 463 bp downstream of the "a" sequence. The LD₅₀ value of 2616 on intracerebral inoculation is >10⁶ PFU/mouse.

### 2621

This virus has a stop codon inserted only in the RL1 open reading frame 9 bp downstream of the initiating methionine within exon 1. The virus has a LD₅₀ of >10⁷ PFU/mouse following intracerebral inoculation.

### In vitro studies of HSV-1716 on a Human Malignant Mesothelioma Cell Line.

A human malignant mesothelioma cell line called REN was isolated, characterised, and passaged as previously described by our laboratory. To construct a single step viral growth curve, REN cells were plated on six-well plates at a density of 500,000 cells/well and infected 24 hours later with HSV-1716 at a multiplicity of infection (MOI) of 0.01 (5000 pfu/well). One well was harvested at 0, 6, 12 and 24 hours by cell scraping and collection of the media. The samples were freeze/thawed and titered on Baby Hamster Kidney cell monolayers. A cell viability assay was performed by plating REN cells in 96 well plates at a density 20,000 cells per well. Twenty four hours later the cells were infected with HSV-1716 at MOI's of 0,0.001, 0.01, 0.1. Six wells were infected at each MOI. Three complete 96 well plates were constructed to allow for viability assay at 24, 48 and 72 hours. Viable cell number was assessed by a colorimetric assay (Cell Titer 96% Aqueous Non-radioactive MTT Cell Proliferation Assay; Promega Corporation, Madison, WI) that measures viable cell dehydrogenase activity by absorbance. The present control growth is defined as the ratio of the mean absorbance of six treatment wells at 490nm to the mean absorbance of six untreated matched controls.

### In vivo Studies

A previously described model of human malignant mesothelioma growing in the peritoneal cavity of SCID mice was utilised for all *in vivo* experimentation (Smythe *et al*, 1994a; Smythe *et al*, 1994b).

Briefly, SCID mice were obtained and housed at the animal facilities of the Wistar Institute (Philadelphia, PA). On day 0, animals were injected intraperitoneally with 30 x 10⁶ REN cells in 1 cc of cell culture media. For the tumor burden study treatment animals were given 4x10⁶pfu of HSV-1716 in culture media by intraperitoneal injection on day 14. Control animals received an equivalent volume of culture media.

The animals were examined daily and sacrificed by cervival dislocation on day 28. The amount of tumor burden was assessed using a four-point semiquantative scale which accounts for both gross and microscopic disease. Briefly, animals were assessed for tumor in the following four areas: stomach/pancreas, portal region, retroperitoneum/diaphram, and small bowel mesentary.

On gross examination animals received either a score of 0 if no tumor was present or a score of 1 in each of the four designated areas where gross tumor was seen. If no gross tumor was visible, H & E stained paraffin embedded sections of each organ from the designated area were examined in a blinded fashion by a clinical pathologist. The sections were scored as either 0 for no microscopic tumor or 0.5 if microscopic tumor was present. Thus, the tumor scores ranged for 0 to 4.0. Organs including: brain, heart, lungs, liver, stomach, pancreas, kidney, adrenals, spleen, gonads, small bowell, and diaphram were obtained from each animal. Each organ was divided by thirds with equal samples designated for frozen section, formalin fixation and DNA extraction.

For the initial survival study, 18 animals were injected intraperitoneally with 30 x 10⁶ REN cells in 1 cc of cell culture media (day 0). On day 7, one animal was sacrificed for gross tumor confirmation and the remaining animals were randomized to the treatment group (n=8) and the control group (n=9). Treatment animals received 4 x 10⁶ pfu of HSV-1716 by intraperitoneal injection; control animals received an equal volume of culture media. The animals were checked daily and followed for survival. An identical protocol was followed for the dose response study except the animals were randomized into the control group (n=10), the high dose group (n=10, 4 x 10⁶ pfu HSV-1716), the middle dose group (n=10, 4 x 10⁵ pfu HSV-1716) and the low dose group (n=10, 4 x 10⁴ pfu HSV-1716).

### Histology and Immunohistochemistry

Tissue samples were obtained at necropsy. A portion of each specimen was fixed in 10% neutral buffered formalin overnight, paraffin embedded, sectioned and stained with hematoxylin and eosin for microscopic examination. Immunohistochemical staining for HSV infection was performed on frozen tissue sections with a commercially available polyclonal antibody for cell surface HSV antigens (DAKO, Carpinteria, CA).

### In vivo Dissemination and Restriction Studies

In order to look for dissemination of HSV-1716, we performed PCR looking for the herpes virus thymidine kinase gene (tk) on the collected tissues from-two animals in the tumor burden study. Genomic DNA was obtained by standard phenol/chloroform extraction and amplified by PCR. The PCR primers (5' ATGG CTTT TCGT ACCC CTGC CAT AND 3' GGTA TCGC GCGG GGGG GTA) were designed to span a region of the HSVtk gene generating a 536 bp fragment. Ten microliters of DNA extract from each tissue sample was subjected to 35 cycles of PCR using the tk primers. The tk plasmid as well as DNA brain tissues from an animal infected with wild type HSV-17+ were used as positive controls. PCR products were run on ethidium bromide 1.5% agarose gels and then blotted overnight onto Zeta-Probe GT Blotting Membranes (Bio-Rad Laboratories, Hercules, CA). The membrane was probed using a ³²P-labeled portion of the HSVtk plasmid corresponding to the 536 bp PCR generated tk fragment.

### RESULTS

### HSV-1716 Efficiently Replicates in a Human Malignant Mesothelioma Cell Line and Lyses the Cells In Vitro.

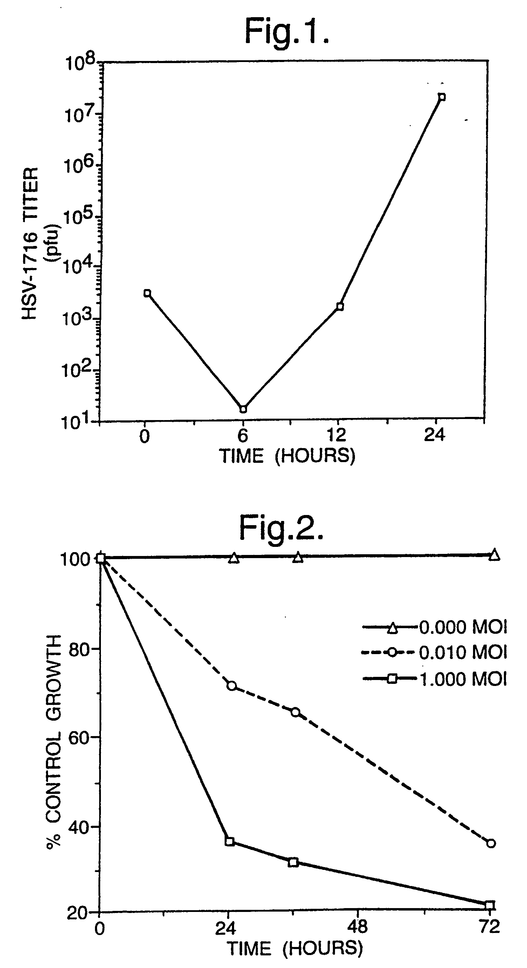

To determine the ability of HSV-1716 to replicate within a non-CNS human tumor cell line in vitro, we performed a single step viral growth curve in REN cells (a human malignant mesothelioma cell line isolated and characterised from a clinical specimen in our laboratory). As shown in Figure 1, REN cells supported rapid growth of the virus. At time 0, 70% of the input viral innoculum was recovered. By 6 hours, the number of recovered active viral particles fell by a factor of 200 as expected due to viral uptake and disassembly in preparation for viral replication. Twelve hours later, viral recovery increased to a level near the input innoculum. By 24 hours, a 4-log increase over the initial innoculum was obtained demonstrating efficient replication of HSV-1716 on REN cells.

To demonstrate the ability of HSV-1716 to -lyse REN cells, we next performed an in vitro target cell viability assay. As shown in Figure 2, HSV-1716 efficiently lysed target cells in a time and dose-responsive fashion. By 72 hours, at an MOI=1.0, less than 20% of the cells remained viable when compared to matched uninfected control cells. Similar results have been obtained with a second human mesothelioma line, I-45 (data not shown).

### Unlike Wild-type HSV-1, HSV-1716 Infection and Replication is Restricted to Tumor Cells in an In vivo SCID Mouse Model of Human Mesothelioma.

As expected, intraperitoneal injection of SCID mice with 5x10⁶ pfu of wild type HSV-17+ led to rapid spread of the virus, neurological dysfunction, and death of all animals by 7 days. To determine the extent of HSV infection, organs from animals sacrificed 72 hours after wild type injection were analysed immunohistochemically with a polyclonal antibody recognising HSV-antigens. Positive cells were clearly seen in the myenteric ganglia of the small intestine, adrenal glands, and brain. In contrast, non-tumor bearing SCID mice injected with the same dose of HSV-1716 remained healthy. Immunohistochemistry for HSV antigens was negative 72 hours after infection.

To test the ability of HSV-1716 to infect and replicate within human tumors *in vivo*, SCID mice were injected intraperitoneally with 30 million human REN cells. After 14 days, diffuse macroscopic 5-8 mm tumor nodules were present. At this time, 5x10⁶ pfu of HSV-1716 were instilled into the peritoneal cavity; 72 hours later, the animals were sacrificed and the abdominal organs processed for immunohistochemistry to detect HSV-proteins. Microscopic examination revealed that virtually all tumor nodules showed necrosis, infiltration with mononuclear inflammatory cells, multinucleated cells and nuclear inclusions consistent with active herpetic infection. In contrast, no viral cytopathic changes were seen in any normal tissues. To directly detect HSV infection, tumors and organs were stained with an anti-HSV antibody. A large percentage of the tumor cells stained positively for HSV antigens while surrounding normal tissues, as well as other normal tissues examined, showed no positive staining. Specifically liver, kidney, spleen, small bowel, myenteric plexuses, adrenal glands, spinal cord and brain were negative. Similar results were obtained at days 5, 7, 9 and 11 after infection, however, the number of positive cells appeared to decrease at the later time points, possibly due to a decrease in available tumor substrate.

### HSV-1716 Does Not Persist Following Intraperitoneal Injection in Tumor-Bearing Mice.

To more sensitively detect the persistence and dissemination of HSV-1716 after intraperitoneal injection, we used the polymerase chain reaction (PCR) to detect the presence of Herpes Simplex Thymidine Kinase (HSVtk) DNA. The results from two tumor-bearing animals demonstrated no HSVtk dissemination and no HSVtk persistence two weeks after intraperitoneal injection. In contrast, one animal who was given HSV-17+ as a positive control, demonstrated HSVtk dissemination to the brain within 72 hours after intraperitoneal injection.

### HSV-1716 reduces intraperitoneal tumor burden and markedly prolongs survival in a SCID mouse model of human mesothelioma.

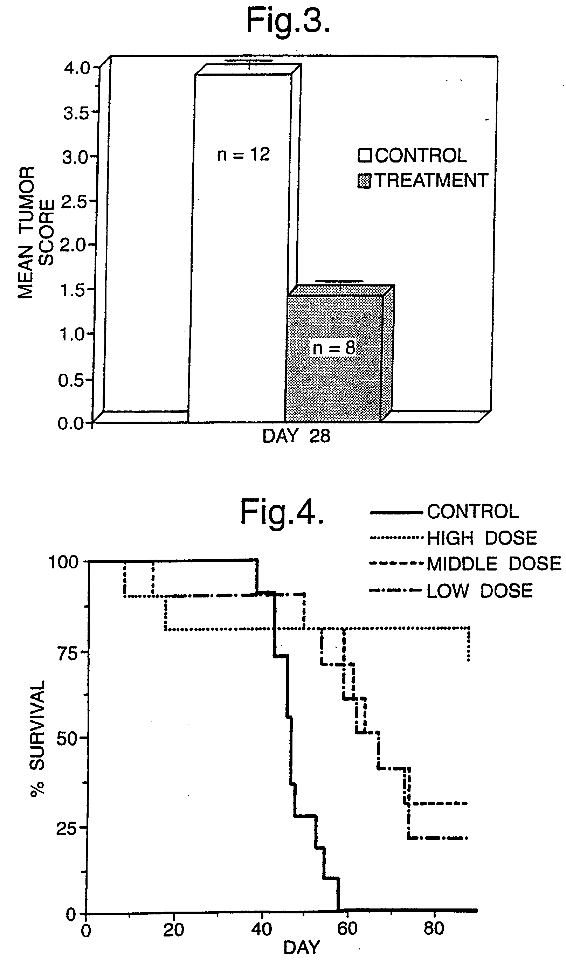

To determine the ability of HSV-1716 infection to erradicate established tumor, 5x10⁶ plaque forming units (pfu) of HSV-1716 were given by intraperitoneal injection to animals that had been injected intraperitoneally 14 days previously with 30 million REN tumor cells. Animals at this time had established intraperitoneal tumors that consisted of multiple 5 to 8 mm nodules with portal invasion and gallbladder distension. Two weeks later, animals were sacrificed and the tumor burden was assessed using a previously developed semi-quantitative scale which accounts for both gross and microscopic tumor (Hwang *et al*, 1995). The tumor score ranges from 0 (no gross or microscopic tumor) to a maximum score of 4.0. Figure 3 shows a significant reduction in the mean tumor score at day 28 in tumor-bearing animals (n=12) treated with HSV-1716 as compared to the mean tumor score in control animals (n=8). The mean tumor score in the treatment group was 1.4 ± 0.2 compared with a mean tumor score in the control group of 3.9 ± 0.1 (p,0.001). All animals in the control group survived the study period. There was one death in the treatment group that occurred 5 days after viral administration. Gross examination failed to reveal the cause of death; microscopic examination was not possible due to autolysis.

To determine if this decrease in tumor mass conferred a survival advantage to SCID mice bearing established intraperitoneal REN tumors, a second set of tumor-bearing animals were injected with 5x10⁶ pfu of HSV-1716 two weeks after intraperitoneal injection of tumor cells and followed for survival. The median survival was increased from 47 days in the control group (n=9) to 95 days in the treatment group (n=10). After 102 days, the remaining 3 surviving animals were sacrificed and necropsied. All deaths in the control group were a result of bulky intraperitoneal disease; no external tumor nodules were visible at the initial tumor injection site. Interestingly, deaths in the treatment group occurred at two distince time points. Three animals died shortly after HSV-1716 administration. There was no evidence of bulky disease at this time. The majority of the other animals died around day 100 due to bulky malignant disease that extended from large subcutaneous nodules arising on the anterior abdominal wall. These nodules corresponded to the site of the initial tumor injection and the tumor appeared to be growing inward from the anterior abdominal with invasion into the peritoneal cavity. There were no deaths in a cohort of non-tumor bearing animals (n=5) who received the same dose of HSV-1716 by intraperitoneal injection.

A second survival study was performed to determine the viral dose response. Tumor bearing animals were randomised to control (n=11) and treatment groups (low dose - 4x10⁴ pfu HSV-1716, n=10; middle dose - 4x10⁵ pfu HSV-1716, n=10; and high dose - 4x10⁶ pfu HSV-1716, n=10). As shown in Figure 4, treatment with high dose HSV-1716 significantly improved survival when compared to control animals (p=0.0011 by Mantel-Cox logrank test). Seventy percent of the high dose animals were alive at day 90; however, 5 out of the 7 developed subcutaneous tumor nodules on the anterior abdominal wall corresponding to the initial tumor injection site. The low and middle dose treatment animals also demonstrated a significant improvement in survival when compared to the control animals (p=0.0003 for control vs. middle dose and p=0.0019 for control vs. low dose by Mantel-Cox logrank test). There was no difference in survival between the low and middle dose animals (p=0.65).

### DISCUSSION OF RESULTS

These results demonstrate that the mutant "replication-restricted" Herpes Simplex Virus-1716 can reduce tumor burden and significantly prolong survival in an animal model of non-CNS localised human malignancy. Furthermore, we have shown that the HSV-1716 mutant is "replication-restricted" to malignant cells, in that it does not disseminate or persist after intraperitoneal injection into SCID mice bearing human tumors. These findings suggest that this virus is efficacious and safe for use in localised human malignancies of non-neuronal origin such as malignant mesothelioma, ovarian carcinoma, or bladder cancer.

The "replication-restriction" of HSV-1716 is provided by deletion of the γ34.5 gene. The function of this gene is still unclear, however, the loss of the γ34.5 gene has been shown correlate with a loss of neurovirulence.²⁴ There are also likely additional functions of this gene that allow for the restricted replication seen in our malignant mesothelioma cell lines.

### EXAMPLE 2

### MATERIALS AND METHODS

### Studies of HSV-1716 on a Human Melanoma Cell Line

The use of replication restricted HSV-1716 to treat experimental human malignant melanoma outside the CNS was investigated as follows. Tests were carried out on mice *in vivo.*

### Tumor Cells:

Human melanoma cell lines were a generous gift from Meenhard Herlyn (Wistar Institute, Phila, PA). Line 1205 was isolated as previously reported (Juhasz *et al*., 1993) and the other 25 lines were isolated in a similar fashion. Cells were grown in plastic flasks in AUTO-POW media containing penicillin, streptomycin, and 5% calf serum at 37°C in a humidified environment with 5% CO₂.

### Virus Production:

To produce virus stocks, subconfluent monolayers of baby hamster kidney 21 clone 13 (BHK) cells were infected with HSV strains 1716 or wild type 17+. Virus was concentrated from the culture and titrated by plaque assay (Spivack & Fraser, 1987). All viral stocks are stored frozen at -80°C, and thawed rapidly just prior to use.

### Production of UV Inactivated Virus:

Ultraviolet inactivation of HSV was performed using the method of Notarianni and Preston (Notarianni & Preston, 1982). After inactivation, the viral suspension was titered on BHK cells to confirm that it could no longer establish a lytic infection, stored frozen at -80°C, and thawed rapidly just prior to use.

### Viral Growth Kinetics Assay

Subconfluent monolayers of 1205 cells (5x10⁵ cells in six well plastic tissue culture plates) were infected with 5x10³ PFU of HSV-1716 in 1 ml of AUTO-POW media containing penicillin, and streptomycin. For the time O measurement the cell monolayer was scrapped off into the viral inoculum suspension immediately, and frozen at -80°C. For the remaining time points, the viral inoculum was incubated on the cell monolayer at 37°C for one hour with gentle rocking, and then aspirated off. The infected monolayers were washed twice with media, and resuspended in 1 ml of AUTO-POW media containing penicillin, streptomycin, and 5% calf serum. At the appropriate times post infection, the monolayers were harvested with a cell scraper, and the suspension frozen at -80°C. Following 2 cycles of freezing and thawing, each sample was titrated by plaque assay on BHK cells (Spivack & Fraser, 1987).

### Intracutaneous Tumor Production and Assays:

Mice were anesthetized with intramuscular ketamine/xylazine (87mg/kg ketamine/13mg/kg xylazine). A patch of hair was removed from one flank using a chemical depilatory agent (Magic Shaving Powder, Carson Products Co., Savannah, GA) Intracutaneous injection of 1x10⁵ 1205 melanoma cells in a total volume of 50µL was performed using a Hamilton syringe, and a disposable 28 g needle. Tumor volumes were calculated based on a radius obtained from orthogonal measurements of tumor diameters using a micrometer-caliper, and assume a spherical tumor shape. On day 14 post tumor cell injection mice were randomly divided into three groups. One group was treated with a 25µl intratumoral injection of 2.5x10⁶ PFU of HSV-1716 using a Hamilton syringe, and a disposable 30g needle. The second and third groups were injected respectively with an equal volume of UV-inactivated HSV-1716 or viral culture medium alone as comparisons.

### Immunohistochemistry:

Viral antigen expressing cells were detected by the indirect avidin-biotin immunoperoxidase method (Vector Labs, Burlingam, CA) as specified by the manufacturer with slight modifications developed in our laboratory (Gesser *et al*., 1994). Rabbit antiserum to HSV-1 (Dako Corp., Carpinteria, CA) was used at a dilution of 1:1000.

### Statistical Analysis:

Data analysis including calculations of means, standard deviations and ANOVA was performed using StatView statistical software (Abacus Concepts, Berkeley CA) on an Apple MacIntosh computer (Cupertino, CA).

### RESULTS

### HSV-1716 and HSV-17+ replicate efficiently in human melanoma cells.

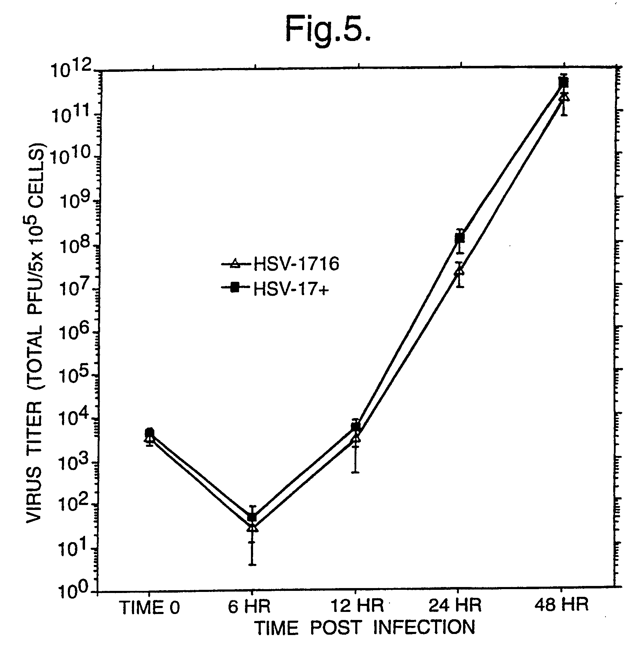

Subconfluent monolayers of 1205 cells were infected with 5x10³ PFU of HSV-1716 or HSV17+. For the time O measurement the cell monolayer was scrapped off into the viral inoculum suspension immediately, and frozen. For the remaining time points, the viral inoculum was incubated on the cell monolayer at 37°C for one hour with gentle rocking, and then aspirated off. The infected monolayers were washed twice with media, and resuspended in 1 ml of AUTO-POW media containing penicillin, streptomycin, and 5% calf serum. At the appropriate times post infection the monolayers were harvested with a cell scraper, and the suspension frozen at -80°C. Following 2 cycles of freezing and thawing, each sample was titrated by plaque assay on BHK cells. The results are shown in Figure 5. The data shown represent the mean ± standard deviation of triplicate determinations at each time point.

### Replication of HSV-1716 is restricted to Melanoma Cells following injection into pre-formed intracutaneous tumor nodules (data not shown).

Intracutaneous tumors were produced by injection of 1x10⁵ 1205 melanoma cells into SCID mice. On day 14 post tumor cell injection, some mice were treated with an intratumoral injection of 2.5 x 10⁶ PFU of HSV-1716, while control mice received an equivalent volume of UV inactivated virus. Sections were taken from a control tumor treated with UV inactivated 1716, and harvested 10 days later. Sections were also taken from a tumor treated with HSV-1716, and harvested 5 days later; and harvested 10 days later. The tumor and surrounding skin were excised, fixed, stained immunohistochemically for HSV-1, and counter stained with hematoxylin.

### Intratumoral Treatment of pre-formed intracutaneous melanoma with HSV-1716 causes a significant inhibition of tumor progression.

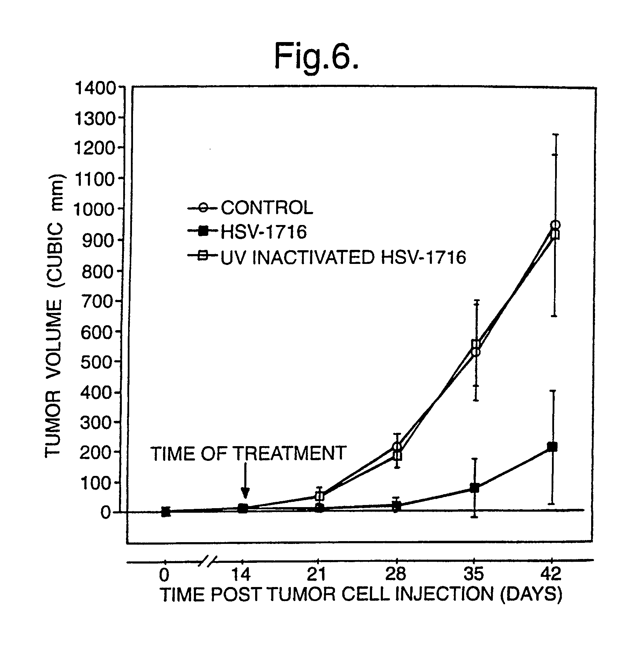

Anesthetised mice were injected intracutaneously with 10⁵ melanoma cells. On day 15 post tumor cell injection, mice were randomly divided into three groups. One group was treated with a 25µl intratumoral injection of 2x10⁶ PFU of HSV-1716, and animals in the two control groups were injected with either an equal volume of UV-inactivated 1716, or viral culture medium. The results are shown in Figure 6. The data shown represent the mean ± standard deviation of 10 mice at each point.

### DISCUSSION OF RESULTS

As an initial step in assessing the viability of HSV based therapy of human intracutaneous melanoma, the efficiency of HSV-1716 replication was determined for human melanoma cell line 1205, and compared to the replication of HSV-17+, the parental strain from which HSV-1716 was derived. As shown in Figure 5, the replication cycle of HSV-1716 in these cells is approximately 24 hrs, and each replication cycle yields an approximately 4 log fold increase in lytically active virus. The kinetics of virus production were nearly identical for HSV-1716 and HSV-17+. An additional 25 human melanoma cell lines were tested for lytic replication of HSV, and all of these replicated HSV-1716 and HSV-17+ efficiently in vitro (data not shown).

We next evaluated whether replication of HSV-1716 could be demonstrated *in vivo* in pre-formed intracutaneous human melanoma nodules growing in SCID mouse skin, and importantly we evaluated if replication of the virus was detectable in surrounding normal tissues. This was accomplished by immunohistochemical evaluation of treated tumors and surrounding tissues. Immunohistochemical staining of intracutaneous 1205 melanoma nodules with antibody to HSV at 5 days or 10 days after intratumoral injection of HSV-1716 demonstrated positive staining that was dispersed throughout a large area of the tumor. At these times a significant portion of the HSV-1716 treated tumor nodules were necrotic. Moreover, no staining was seen in normal murine tissues surrounding the tumor nodules in these and all other sections examined. No staining, and no significant necrosis was seen in the representative control tumor treated with UV-inactivated HSV-1716 and subjected to the full immunohistochemical protocol.

After determining that HSV-1716 exhibits restricted replication within intracutaneous melanoma, we performed intratumoral therapy on pre-formed tumors, to test the efficacy of this approach. Figure 6 shows the tumor volume of treated and control tumors at various times after viral therapy, and demonstrates that HSV-1716 significantly inhibits progression of pre-formed intracutaneous melanoma. UV inactivated HSV-1716 had no effect on tumor progression relative to nodules injected with viral culture medium alone. Of the ten tumor bearing mice treated with HSV-1716, three had complete involution of melanoma nodules such that no palpable tumors were present from day 21 on. ANOVA analysis of the tumor volume data demonstrated that the effect of HSV-1716 treatment is statistically significant (p<0.0001) at all times post treatment examined. In none of the HSV-1716 treated animals was there any mortality or morbidity noted.

It is therefore apparent from Figure 6 that HSV-1716 has the ability to reduce melanoma tumor size *in vivo* in mice into which melanoma tumors have been introduced by intracutaneous injection.

### REFERENCES

Gesser *et al*. Restricted herpes simplex virus type 1 gene expression within sensory neurons in the absence of functional B and T lymphocytes. Virology 200, 791-795, 1994.

Hwang *et al*. Gene Therapy using adenovirus carrying the Herpex Simplex-Thymidine Kinase Gene to treat *in vivo* models of human malignant mesothelioma. Am. J. Respir. Cell Mol. Biol. 13, 7-16, 1995.

Juhasz *et al*. Growth and invasion of human melanomas in human skin grafted to immunodeficient mice. Amer.J. of Path. 143, 528-37, 1993.

Markert *et al*. Reduction and elimination of encephalitis in an experimental glioma therapy model with attenuated herpes simplex mutants that retain susceptibility to acyclovir. Neurosurgery 32, 597-603, 1993.

Martuza *et al.* Experimental therapy of human glioma by means of a genetically engineered virus mutant. Science 252, 854-856, 1991.

Mineta *et al*. Treatment of malignant gliomas using a ganciclovir hypersensitive, ribonucleotide reductase-deficient herpes simplex viral mutant. Cancer Res. 54, 3963-3966, 1994.

Mineta *et al.* Attenuated multi-mutated herpes simplec virus-1 for the treatment of malignant gliomas. Nature Medicine 1, 938-943, 1995.

Notarianni and Preston. Activation of cellular stress protein genes by herpes simplex virus temperature-sensitive mutants which overproduce immediate early polypeptides. Virology 123, 113-122, 1982.

Smythe *et al* (1994a). Use of recombinant adenovirus to transfer the herpes simplex thymidine kinase (HSVtk) gene to thoracic neoplasms: an effective in vitro drug sensitization system. Cancer Res. 54, 2055-2059, 1994.

Smythe *et al* (1994b). Treatment of experimental human mesothelioma using adenovirus transfer of the Herpes Simplex-kinase gene. Ann Surg 222, 78-86, 1994.

Spivack and Fraser. Detection of herpes simplex type 1 transcripts during latent infection in mice. J. Virol. 61, 3841-3847, 1987.

## Claims

1. Use of a mutant herpes simplex virus which has been modified in the γ34.5 gene such that the gene is non-functional, in the manufacture of a medicament for use in treating a non-neuronal cancer.

2. Use according to claim 1, wherein the medicament is for use in treating a human with a non-neuronal cancer.

3. Use according to claim 1 or 2, wherein the cancer is a primary tumor.

4. Use according to claim 1 or 2, wherein the cancer is a metastatic tumor.

5. Use according to claim 1 or 2, wherein the cancer is a mesothelioma, ovarian carcinoma, bladder cancer or melanoma.

6. Use according to any one of the preceding claims, wherein the mutant herpes simplex virus is a type 1 herpes simplex virus.

7. Use according to any one of the preceding claims, wherein the mutant herpes simplex virus has been modified within the BamHI restriction fragment of the long terminal repeat of the viral genome.

8. Use according to claim 7, wherein the modification is a deletion of from 0.1 to 3kb.

9. Use according to claim 8, wherein the deletion is from 0.7 to 0.8kb.

10. Use according to any one of the preceding claims, wherein the mutant herpes simplex virus is strain 1716.

11. A method of treating a non-neuronal cancer in a mammal, which method comprises the step of administering to the mammal an effective amount of a mutant herpes simplex virus which has been modified in the γ34.5 gene such that the gene is non-functional.

12. An agent for treating a non-neuronal cancer, comprising a mutant herpes simplex virus which has been modified in the γ34.5 gene such that the gene is non-functional.
